(19) Europäisches Patentamt
European Patent Office
Office européen des brevets

(11) Numéro de publication: **0 082 782**
**B1**

(12) # FASCICULE DE BREVET EUROPEEN

(45) Date de publication du fascicule du brevet: **20.03.85**

(21) Numéro de dépôt: **82402327.9**

(22) Date de dépôt: **17.12.82**

(51) Int. Cl.⁴: **C 07 C 43/313**, C 07 C 47/24, C 07 D 319/06, C 07 C 41/56, C 07 C 45/63

(54) **Procédé de préparation d'halogénoacétals éthyléniques.**

(30) Priorité: **18.12.81 FR 8123685**

(43) Date de publication de la demande:
**29.06.83 Bulletin 83/26**

(45) Mention de la délivrance du brevet:
**20.03.85 Bulletin 85/12**

(84) Etats contractants désignés:
**AT BE CH DE FR GB IT LI LU NL SE**

(56) Documents cités:
**DE - A - 2 708 281**
**DE - A - 2 708 304**
**FR - A - 1 371 230**

**JOURNAL OF THE CHEMICAL SOCIETY, PERKIN TRANSACTIONS I, ORGANIC AND BIO-ORGANIC CHEMISTRY, 1981, pages 2411-2414;**
**TETRAHEDRON LETTERS, no. 6, février 1971, pages 493-494, Pergamon Press, (GB); M.J. BERENGUER et al.: "gamma-Halogenation of alpha, beta-unsuturated aldehydes".**

(73) Titulaire: **RHONE-POULENC SANTE, Les Miroirs 18 Avenue d'Alsace, F-92400 Courbevoie Cedex (FR)**

(72) Inventeur: **Desmurs, Jean, 49 avenue Lacassagne, F-69003 Lyon (FR)**

(74) Mandataire: **Pilard, Jacques et al, RHONE-POULENC RECHERCHES Service Brevets Pharma 25, Quai Paul Doumer, F-92408 Courbevoie Cedex (FR)**

**EP 0 082 782 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un nouveau procédé de préparation d'halogénoacétals éthyléniques de formule générale:

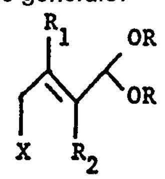

(I)

dans laquelle X représente un atome d'halogène choisi parmi le chlore et le brome, les symboles $R_1$ et $R_2$, identiques ou différents, représentent un atome d'hydrogène ou un radical alcoyle contenant 1 à 6 atomes de carbone, et les symboles R représentent chacun un radical alcoyle droit ou ramifié contenant 1 à 6 atomes de carbone ou forment ensemble un radical alcoylène droit ou ramifié contenant 2 à 6 atomes de carbone.

Les halogénoacétals éthyléniques de formule générale (I) sont des composés organiques particulièrement utiles en synthèse organique. Ils peuvent être utilisés pour introduire un motif aldéhydique, α,β-éthylénique sur un reste mono- ou polyénique par réaction avec une sulfone éthylénique ou polyénique en présence d'un agent alcalin selon le procédé décrit dans le brevet belge N° 794872, la sulfone résultant de cette condensation étant ensuite désulfonée avec formation d'une double liaison supplémentaire.

En particulier, le rétinal (aldéhyde de la vitamine A) peut être préparé par action du bromo-4 méthyl-3 diéthoxy-1,1 butène-2 sur la phényl(triméthyl-2,6,6 cyclohexén-1 yl)-5 méthyl-3 pentadién-2,4 ylsulfone, puis désulfonation du phénylsulfonyl-5 (triméthyl-2,6,6 cyclohexén-1 yl)-9 diéthoxy-1,1 diméthyl-3,7 nonatriène-2,6,8 al-1 ainsi obtenu en rétinal.

Il est connu que les γ-halogénoacétals d'aldéhydes α,β-éthyléniques peuvent être préparés par halogénoalcoylation d'un alcoyloxy-1 diène-1,3 par action d'un N-halogénosuccinimide en présence d'un alcool suivant le procédé décrit par S.M. Makin et coll., «J. Gen. Chem. U.R.S.S.», *32*, 1088 (1962). Ce procédé a pour inconvénient la difficulté d'accès aux éthers diéthyléniques de départ qui sont généralement préparés par traitement de trialcoxy-1,1,3 méthyl-3 butane et de dialcoxy-1,1 méthyl-3 butène-2 à haute température en présence de catalyseurs [S.M. Makin et coll., «J. Gen. Chem. U.R.S.S.», *29*, 116 (1959)], l'accès à ces motifs étant également difficile.

Il est connu également, d'après les brevets belges N$^{os}$ 851779 et 851780, de préparer les produits de formule générale (I) par action d'un agent d'halogénation choisi parmi les cations halogènes $C^+$, $Br^+$ et $I^+$ soit sur un ester de formule générale:

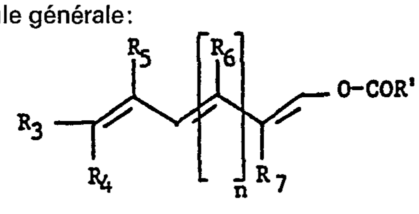

(II)

dans laquelle R' représente un radical alcoyle contenant 1 à 6 atomes de carbone en chaîne droite ou ramifiée, $R_3$, $R_4$, $R_5$, $R_6$ et $R_7$, identiques ou différents, représentent un atome d'hydrogène ou un radical alcoyle contenant 1 à 6 atomes de carbone en chaîne droite ou ramifiée, ou alcényle contenant 3 à 6 atomes de carbone en chaîne droite ou ramifiée et dont la double liaison est en position autre que 1-2, et n est égal à 0, 1, 2, 3 ou 4, étant entendu que, lorsque n est supérieur à 1, les différents symboles $R_6$ peuvent être identiques ou différents, soit sur un énoxysilane de formule générale:

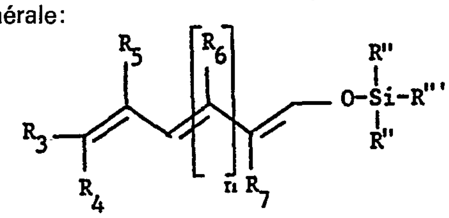

(III)

dans laquelle $R_3$, $R_4$, $R_5$, $R_6$ et $R_7$ sont définis comme ci-dessus, R'' représente un radical alcoyle contenant 1 à 12 atomes de carbone en chaîne droite ou ramifiée, cycloalcoyle contenant 3 à 6 atomes de carbone, phényle, alcoylphényle dont la partie alcoyle contient 1 à 6 atomes de carbone ou phénylalcoyle dont la partie alcoyle contient 1 à 6 atomes de carbone et R''' est identique à R'' ou représente un reste de formule générale:

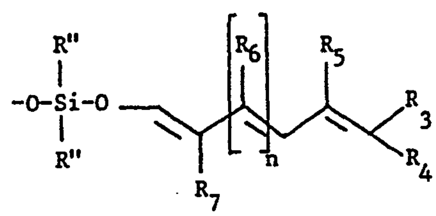

(IV)

dans laquelle $R_3$, $R_4$, $R_5$, $R_6$, $R_7$, n et R'' sont définis comme ci-dessus, puis par action d'un alcool aliphatique primaire ou secondaire ou d'un glycol. Parmi les sources de cations halogènes peuvent, entre autres, être cités les complexes fournis par action du chlore, du brome ou de l'iode sur le diméthylformamide, le diméthylacétamide ou la N-méthylpyrrolidone.

Les procédés connus dans l'état antérieur de la technique présentent l'inconvénient de mettre en œuvre des réactifs chers tels que l'acétate d'isoprényle ou le triméthylchlorosilane pour transformer le prénal en halogénoacétal de formule générale (I).

Il a maintenant été trouvé, et c'est ce qui fait l'objet de la présente invention, que les halogénoacétals de formule générale (I) peuvent être obtenus en faisant réagir le chlore ou le brome en présence d'un amide tertiaire tel que le diméthylformamide, le diméthylacétamide ou la N-méthylpyrrolidone sur un aldéhyde β,γ-éthylénique pour obtenir l'halogénoaldéhyde de formule générale:

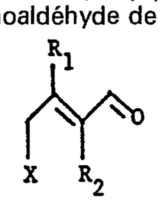

(V)

dans laquelle X représente un atome de chlore ou de brome et $R_1$ et $R_2$ sont définis comme précédemment, qui est transformé en halogénoacétal de formule générale (I) par action d'un alcool aliphatique primaire ou secondaire, d'un glycol ou d'un orthoformiate d'alcoyle.

D'une manière générale, pour préparer l'halogénoaldéhyde de formule générale (V), il est suffisant de faire réagir une molécule d'halogène par mole d'aldéhyde de formule générale:

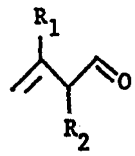

(VI)

dans laquelle $R_1$ et $R_2$ sont définis comme précédemment, un excès de l'un ou l'autre de ces réactifs pouvant toutefois être utilisé sans inconvénient.

Généralement, l'halogénation de l'aldéhyde de formule générale (VI) est effectuée à une température comprise entre −40 et 0°C.

L'acétalisation de l'halogénoaldéhyde de formule générale (V) peut être effectuée:

– soit en faisant réagir un alcool aliphatique primaire ou secondaire, un glycol ou un orthoformiate d'alcoyle en présence d'un acide minéral tel que l'acide chlorhydrique ou bromhydrique sur l'halogénoaldéhyde isolé du mélange réactionnel à la fin de la phase d'halogénation,

– soit en ajoutant un alcool aliphatique primaire ou secondaire, un glycol ou un orthoformiate d'alcoyle au mélange réactionnel à la fin de la phase d'halogénation.

L'acétalisation s'effectue généralement à une température comprise entre 0 et 50°C.

L'halogénoacétal éthylénique de formule générale (I) obtenu selon le procédé de la présente invention peut être isolé du mélange réactionnel et purifié par application des méthodes habituelles d'extraction et de purification (distillation, chromatographie).

L'aldéhyde de formule générale (VI) peut être obtenu selon les méthodes habituelles. Par exemple, le méthyl-3 butène-3 utilisé comme matière première peut être préparé selon la méthode décrite par J.W. Cornforth et F.P. Ross, «Chemical Communications», 1395 (1970) par hydrolyse du diéthoxy-1,1 méthyl-3 butène-3 qui est obtenu par action du chlorure de métallyle sur l'orthoformiate d'éthyle en présence de magnésium.

Le procédé selon la présente invention permet d'obtenir les halogénoacétals de formule générale (I) avec de bons rendements à partir de matières premières plus facilement accessibles que celles utilisées dans les procédés antérieurement connus.

Les exemples suivants, donnés à titre non limitatif, illustrent la mise en œuvre de la présente invention.

*Exemple 1:*

Dans un réacteur de 500 $cm^3$ muni d'un agitateur, d'un réfrigérant et d'un tube plongeant permettant l'introduction du chlore, on introduit, après avoir purgé préalablement par un courant d'argon, 6,08 g de méthyl-3 butène-3 al (0,0687 mol) et 80 $cm^3$ de diméthylformamide. Après avoir refroidi le mélange à −20°C, on introduit, en 70 min, 4,88 g de chlore gazeux (0,0685 mol) entraîné par un courant d'argon. Après la fin de l'addition du chlore, l'agitation est poursuivie pendant 40 min à une température de −20°C. Après avoir laissé la température remonter au voisinage de 20°C, on verse le mélange réactionnel dans 170 $cm^3$ d'eau glacée saturée de chlorure de sodium. Après extraction du mélange par 2 fois 100 $cm^3$ d'éther diéthylique, la phase organique est lavée par 50 $cm^3$ d'eau saturée de chlorure de sodium, séchée sur sulfate de sodium puis concentrée à sec sous pression réduite (20 mm de mercure; 2,7 kPa) à 20°C. On obtient ainsi 10,22 g d'une huile jaune qui contient, d'après le dosage par résonance magnétique nucléaire, 60% de chloro-4 méthyl-3 butène-2 al.

Le méthyl-3 butène-3 al, utilisé comme produit de départ, peut être préparé de la manière suivante:

Dans un réacteur de 3 l muni d'une agitation, d'un réfrigérant ascendant et d'une ampoule de coulée, préalablement purgé à l'argon, on introduit 792,3 g d'orthoformiate d'éthyle (5,353 mol) et 346,5 g de magnésium (14,437 atome-gramme). Après avoir chauffé le mélange réactionnel à 60°C, on ajoute 8 $cm^3$ de chlorure de méthallyle et 0,1 g d'iodure de méthyle. Dès que la réaction démarre, on arrête le chauffage puis on ajoute 452,7 g de chlorure de méthallyle (5,002 mol) de façon à maintenir la température au voisinage de 60°C. L'addition dure environ 14 h. Après refroidissement à une température voisine de 5°C, on ajoute, sans que la température dépasse 10°C, 400 $cm^3$ d'une solution saturée de chlorure d'ammonium.

Après avoir ajouté 800 $cm^3$ d'éther diéthylique, les composés minéraux sont éliminés par filtration et lavés avec 2 fois 1000 $cm^3$ d'éther diéthylique. Le filtrat et les lavages sont repris par 500 $cm^3$ d'eau contenant 31 g d'acide oxalique afin d'éliminer l'orthoformiate d'éthyle qui n'a pas réagi. La phase organique est lavée avec 2 fois 50 $cm^3$ d'une solution saturée de carbonate de potassium puis est séchée sur sulfate de sodium. Après filtration et concentration à sec sous pression réduite (200 mm de mercure; 27 kPa) à 30°C, on obtient 731 g d'un liquide incolore qui est purifié par distillation. On obtient ainsi 646,9 g de diéthoxy-1,1 méthyl-3 butène-3 (P.E. $_{2,13\ kPa}$ = 57°C) titrant 95%.

A une solution de 7,6 g d'acide oxalique dihydraté dans 1000 $cm^3$ d'eau, on ajoute 199,9 g de diéthoxy-1,1 méthyl-3 butène-3 (1,2019 mol). La suspension est agitée pendant 6 h 50 min. Le mélange est extrait par 5×200 $cm^3$ de pentane. La phase organique, séchée sur sulfate de sodium, est concentrée rapidement à sec sous pression réduite à une température inférieure à 30°C de façon à éliminer les traces d'acide oxalique. La phase pentanique est alors reprise pour être distillée dans une colonne de 40 cm à garnissage Mutiknit. On

obtient ainsi 53,5 g de méthyl-3 butène-3 al-1 (P.E. $_{101,3\,kPa}$ = 93-93,5°C) titrant 95%.

*Exemple 2:*

Dans un réacteur de 500 cm³, muni d'une agitation, d'un réfrigérant ascendant et d'un tube plongeant permettant l'introduction du chlore, on introduit, après avoir préalablement purgé à l'argon, 9,30 g de méthyl-3 butène-3 al (0,1009 mol) et 100 cm³ de diméthylformamide préalablement distillé et séché. Après refroidissement de la solution à −20°C, on introduit, en 90 min, 7,17 g de chlore gazeux (0,1007 mol) entraîné par un courant d'argon.

L'agitation est maintenue pendant 30 min à −20°C après la fin de l'addition du chlore. On laisse ensuite la température remonter au voisinage de 20°C, puis on ajoute 300 cm³ de méthanol. Le mélange réactionnel est agité pendant 2 h 45 min puis il est versé dans une solution de 12,7 g de bicarbonate de sodium dans 370 cm³ d'eau. La phase aqueuse, saturée en chlorure de sodium, est extraite par 200 cm³ puis 4×100 cm³ d'éther diéthylique. La phase organique est lavée successivement par 2×50 cm³ d'eau et 50 cm³ d'une solution saturée de bicarbonate de sodium, puis elle est séchée sur sulfate de sodium. Après filtration, la phase organique est concentrée à sec sous pression réduite (20 mm de mercure; 2,7 kPa). On obtient ainsi 15,93 g d'une huile orange dans laquelle on dose, par résonance magnétique nucléaire, 65% de chloro-4 diméthoxy-1,1 méthyl-3 butène-2 soit 19,5% d'isomère cis et 45,5% d'isomère trans.

Le rendement de la réaction est de 62,4%.

*Exemple 3:*

Dans un réacteur de 500 cm³, muni d'un agitateur, d'un réfrigérant ascendant et d'un tube plongeant permettant l'introduction du chlore, préalablement purgé à l'argon, on introduit 12,55 g de méthyl-3 butène-3 al (0,136 mol) et 165 cm³ de diméthylformamide préalablement distillé et séché. Après avoir refroidi cette solution à −40°C, on introduit, en 1 h 30 min, 9,68 g de chlore gazeux (0,136 mol) entraîné par un courant d'argon. L'agitation est poursuivie pendant 30 min après la fin de l'addition puis on laisse la température remonter au voisinage de 20°C. On ajoute alors, en 15 min, 14,61 g de diméthyl-2,2 propanediol-1,3 (0,136 mol) en solution dans 30 cm³ de diméthylformamide. La solution est agitée pendant 1 h 15 min puis elle est versée dans une solution de 17,1 g de bicarbonate de sodium dans 500 cm³ d'eau. On extrait alors par 4 fois 100 cm³ d'éther diéthylique. La phase organique est lavée par 2×50 cm³ d'eau, séchée sur sulfate de sodium puis concentrée à sec. On obtient ainsi 26,9 g d'un liquide orangé dans lequel on dose, par résonance magnétique nucléaire, 89% de (chloro-3 méthyl-2 propén-1 yl-1)-2 diméthyl-4,4 dioxanne-1,3.

*Exemple 4:*

Dans un réacteur de 500 cm³, muni d'un agitateur, d'un réfrigérant ascendant et d'une ampoule de coulée, préalablement purgé à l'argon, on introduit 9,22 g de méthyl-3 butène-3 al (0,1 mol) et 100 cm³ de N-méthylpyrrolidone distillée et séchée. Après avoir refroidi cette solution à −10°C, on ajoute, en 1 h 30 min, 15,98 g de brome (0,0999 mol). L'agitation est maintenue pendant 30 min à −10°C puis on laisse la température remonter au voisinage de 20°C. On ajoute alors, en 15 min, 300 cm³ d'éthanol. Le mélange réactionnel est agité pendant 2 h 45 min, puis il est versé dans une solution de 12,7 g de bicarbonate de sodium dans 370 cm³ d'eau. On extrait par 300 cm³ puis 2×100 cm³ d'éther diéthylique. La phase organique est lavée par 2×50 cm³ d'eau puis elle est séchée sur sulfate de sodium. Après filtration et évaporation de l'éther, on obtient 15,92 g d'un liquide marron dans lequel on dose, par résonance magnétique nucléaire, 70% de bromo-4 diéthoxy-1,1 méthyl-3 butène-2.

## Revendications

1. Procédé de préparation d'halogénoacétals éthyléniques de formule générale:

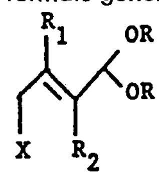

dans laquelle X représente un atome d'halogène choisi parmi le chlore et le brome, les symboles $R_1$ et $R_2$, identiques ou différents, représentent un atome d'hydrogène ou un radical alcoyle contenant 1 à 6 atomes de carbone et les symboles R représentent chacun un radical alcoyle droit ou ramifié contenant 1 à 6 atomes de carbone ou forment ensemble un radical alcoylène droit ou ramifié contenant 2 à 6 atomes de carbone, caractérisé en ce que l'on fait réagir le chlore ou le brome en présence d'un amide tertiaire sur un aldéhyde β,γ-éthylénique pour obtenir l'halogénoaldéhyde de formule générale:

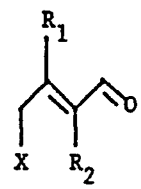

dans laquelle X, $R_1$ et $R_2$ sont définis comme précédemment, puis on fait réagir un alcool aliphatique primaire ou secondaire contenant 1 à 6 atomes de carbone ou un glycol contenant 1 à 6 atomes de carbone ou un orthoformiate d'alcoyle, et isole le produit obtenu.

2. Procédé selon la revendication 1, caractérisé en ce que l'amide est choisi parmi le diméthylformamide, le diméthylacétamide ou la N-méthylpyrrolidone.

3. Procédé selon la revendication 1, caractérisé en ce que la phase d'halogénation est effectuée à une température comprise entre −40 et 0°C.

4. Procédé selon la revendication 1, caractérisé en ce que la réaction d'acétalisation est effectuée à une température comprise entre 0 et 50° C.

5. Procédé selon la revendication 1, caractérisé en ce que la réaction d'acétalisation est effectuée en présence d'un acide minéral sur l'halogénoaldéhyde isolé du mélange réactionnel à la fin de la phase d'halogénation.

6. Procédé selon la revendication 1, caractérisé en ce que la réaction d'acétalisation est effectuée directement sur le mélange réactionnel à la fin de la phase d'halogénation.

## Patentansprüche

1. Verfahren zur Herstellung von äthylenischen Halogenacetalen der allgemeinen Formel:

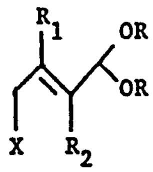

in welcher X ein Halogenatom, ausgewählt aus Chlor und Brom, darstellt, die Symbole $R_1$ und $R_2$, die gleich oder voneinander verschieden sind, ein Wasserstoffatom oder einen Alkylrest mit 1 bis 6 Kohlenstoffatomen darstellen und die Symbole R jeweils einen geradkettigen oder verzweigten Alkylrest mit 1 bis 6 Kohlenstoffatomen darstellen oder zusammen einen geradkettigen oder verzweigten Alkylenrest mit 2 bis 6 Kohlenstoffatomen bilden, dadurch gekennzeichnet, dass man Chlor oder Brom in Gegenwart eines tertiären Amids auf einem β,γ-äthylenischen Aldehyd unter Bildung des Halogenaldehyds der allgemeinen Formel:

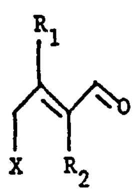

in welcher X, $R_1$ und $R_2$ die obige Bedeutung haben, einwirken lässt und dann einen primären oder sekundären aliphatischen Alkohol mit 1 bis 6 Kohlenstoffatomen oder ein Glykol mit 1 bis 6 Kohlenstoffatomen oder ein Alkylorthoformiat einwirken lässt und das erhaltene Produkt isoliert.

2. Verfahren nach Anspruch 1, dadurch gekennzeichnet, dass das Amid ausgewählt wird aus Dimethylformamid, Dimethylacetamid oder N-Methylpyrrolidon.

3. Verfahren nach Anspruch 1, dadurch gekennzeichnet, dass die Halogenierungsstufe bei einer Temperatur zwischen −40 und 0° C ausgeführt wird.

4. Verfahren nach Anspruch 1, dadurch gekennzeichnet, dass die Acetalisierungsreaktion bei einer Temperatur von 0 bis 50° C ausgeführt wird.

5. Verfahren nach Anspruch 1, dadurch gekennzeichnet, dass die Acetalisierungsreaktion in Gegenwart einer anorganischen Säure an dem aus der Reaktionsmischung am Ende der Halogenierungsstufe isolierten Halogenaldehyd ausgeführt wird.

6. Verfahren nach Anspruch 1, dadurch gekennzeichnet, dass die Acetalisierungsreaktion direkt an der Reaktionsmischung am Ende der Halogenierungsstufe ausgeführt wird.

## Claims

1. Process for the preparation of ethylenic halogenoacetals of the general formula:

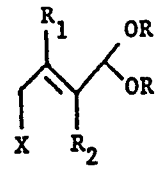

in which X represents a halogen atom chosen from amongst chlorine and bromine, the symbols $R_1$ and $R_2$, which may be identical or different, represent a hydrogen atom or an alkyl radical containing 1 to 6 carbon atoms and the symbols R each represent a straight or branched alkyl radical containing 1 to 6 carbon atoms or together form a straight or branched alkylene radical containing 2 to 6 carbon atoms, characterised in that chlorine or bromine is reacted with a β,γ-ethylenic aldehyde in the presence of a tertiary amide to give the halogenoaldehyde of the general formula:

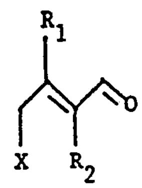

in which X, $R_1$ and $R_2$ are defined as above, after which this is reacted with a primary or secondary aliphatic alcohol containing 1 to 6 carbon atoms or a glycol containing 1 to 6 carbon atoms or an alkyl orthoformate, and the product obtained is isolated.

2. Process according to Claim 1, characterised in that the amide is chosen from among dimethylformamide, dimethylacetamide or N-methylpyrrolidone.

3. Process according to Claim 1, characterised in that the halogenation stage is carried out at a temperature of between −40 and 0° C.

4. Process according to Claim 1, characterised in that the acetalisation reaction is carried out at a temperature of between 0 and 50° C.

5. Process according to Claim 1, characterised in that the acetalisation reaction is carried out, in the presence of an inorganic acid, on the halogenoaldehyde which has been isolated from the reaction mixture at the end of the halogenation stage.

6. Process according to Claim 1, characterised in that the acetalisation reaction is carried out directly on the reaction mixture at the end of the halogenation stage.